(19) Europäisches Patentamt
European Patent Office
Office européen des brevets

(11) **EP 4 703 717 A1**

(12) **EUROPEAN PATENT APPLICATION**

(43) Date of publication:
**04.03.2026 Bulletin 2026/10**

(21) Application number: **24197428.6**

(22) Date of filing: **29.08.2024**

(51) International Patent Classification (IPC):
**G01N 27/30** (2006.01)  **G01N 33/543** (2006.01)

(52) Cooperative Patent Classification (CPC):
**G01N 27/30; G01N 33/5438;** G01N 2600/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Tallinn University of Technology 19086 Tallinn (EE)**

(72) Inventors:
• **SÕRITSKI, Vitali**
  **19086 Tallinn (EE)**
• **REUT, Jekaterina**
  **19086 Tallinn (EE)**
• **BOROZNJAK, Roman**
  **19086 Tallinn (EE)**
• **AYANKOJO, Akinrinade George**
  **19086 Tallinn (EE)**

(74) Representative: **AAA Patendibüroo OÜ Tartu mnt 16 10117 Tallinn (EE)**

(54) **A METHOD FOR A PREPARATION OF A MOLECULARLY-IMPRINTED-POLYMER (MIP) ELECTROCHEMICAL SENSOR FOR AN ELECTROCHEMICALLY INACTIVE ANALYTE DETECTION, A METHOD FOR USING SAID SENSOR AND SUCH A SENSOR**

(57)    The invention relates to a method for a preparation of a molecularly-imprinted-polymer (MIP) electrochemical sensor for an electrochemically inactive analyte detection, a method for using said sensor and such a sensor, where a redox-active surface of the sensor is preferably made of a transition metal oxide, and more preferably ruthenium oxide ($RuO_2$), wherein the redox-active surface is coated by a MIP layer, wherein the MIP layer comprises a plurality of analyte-selective cavities. Said sensor is comprising at least one electrode with an electrically conductive surface with a redox-active surface, preferably a transition metal oxide, and more preferably ruthenium oxide.

FIG 1

★ molecule of a target analyte (e.g. BDNF)
ⓔ electrons

**Description**

TECHNICAL FIELD

Present invention relates to a method for a preparation of a molecularly-imprinted-polymer (MIP) electrochemical sensor for an electrochemically inactive analyte detection. Present invention also relates to a method for using a MIP electrochemical sensor and a molecularly-imprinted-polymer (MIP) electrochemical sensor.
The present invention relates to the field of sensors and more particularly to sensors for detecting an analyte, more particularly to a sensor coated by a molecularly imprinted polymer (MIP).

BACKGROUND ART

European patent application EP3721985A1 (Tallinn Technical University) discloses a molecularly imprinted polymers sensors for sulfonamides and specifically relates to a sensor for the detection of a sulfonamide. The sensor, preferably a Screen Printed Electrochemical sensor (SPE), has a working electrode coated by a Molecularly Imprinted Polymer (MIP) imprinted by a sulfonamide. Said patent application also relates to a method for preparing such a sensor and comprising the steps of polymerization of m-PD on the working electrode of the sensor in presence of the sulfonamide; and extraction of sulfonamide molecules from the polymeric layer thereby obtaining a MIP layer.
Closest prior art is described in the USA patent US11193940B2 (Tallinn Technical University) relating to a sensor for the detection of Neurotrophic Factor (NF). The sensor, preferably a Screen Printed Electrochemical sensor (SPE), has a working electrode coated by a Molecularly Imprinted Polymer (MIP) imprinted by a NF. Said patent also relates to a method for preparing such a sensor and comprising the steps of formation of a cleavable linking layer on the working electrode of the sensor; immobilization of NF molecules on the cleavable linking layer; polymerization of m-PD on the working electrode of the sensor; and cleavage of the cleavable linking layer thereby removing the NF molecules from the MIP layer.

SUMMARY OF INVENTION

**1. Introduction**

The COVID-19 outbreak has underlined the critical need for swift and precise diagnostic tools, bringing rapid evaluation of biological material to the forefront of scientific concern. As a result, research and development in sensing technologies are accelerated to improve their utility in infectious illness diagnosis and monitoring. Consequently, electrochemical sensors that permit accurate and rapid detection of compounds that are of human or environmental concern are playing critical roles in various fields, including clinical diagnostics and environmental monitoring [1].
Pivotal to this are robust artificial receptors which when integrated with appropriate sensor transducers provide sensitive and selective detection of the target molecules.
Notably, molecularly imprinted polymers (MIPs), with their exceptional molecular recognition capabilities, offer a tailored solution as biomimetic receptor layers that selectively bind target molecules [2,3]. Described as a synthetic analogue to natural antibody, MIP operates by a mechanism similar to the "lock-key" for selective capturing of the target [4]. MIP preparation entails the formation of target-specific molecular cavities or moulds within synthetic polymer by templating either the target or a surrogate molecule during the polymer curation. By combining MIPs with modern and sophisticated electrode materials, electrochemical sensing platforms with significantly enhanced sensitivity and efficiency could be easily achieved.
Electropolymerization is a preferred strategy to achieve the seamless and quick integration of a polymer film on an electrode surface in a tunable and cost-efficient manner [5]. Compared to other polymerization methods, electropolymerization avoids the use of exogenous initiators which could be detrimental especially to protein structure and helps to control polymer thickness thereby ensuring reproducibility of the performance of MIP-based sensors [2]. In addition, electropolymerization facilitates rapid transition of MIP-based sensors into electroanalytical devices that is much needed for the achievement of easily commercialized detection system for routine analysis.
Redox probe solutions including ferricyanide/ferrocyanide, ferrocene and its derivatives, etc, are commonly employed in electrochemical characterization of the performance of MIP-based sensors. This is especially critical for electrochemically inactive target molecules whose indirect detection follows the measurement of its modulation of diffusion of redox probe molecules through the porous MIP surface.
Two possible explanations exist for this mechanism including the "gate effect", where the binding of target molecules to specific cavities on the non-conducting MIP causes a swelling or shrinking of the polymer film [6]. This affects diffusion of redox probe ions through the film and is accompanied by a change in the Faradaic current. An alternative mechanism entails the physical obstruction of the electrode surface by the adsorbed electrochemically inactive target molecule thereby preventing the permeability of the redox ion to the electrode surface [7-9].

However, the employment of such external solution presents its own limitations emanating majorly from inadequate mimicking of the real complex environment of the targeted matrix hence, potentially contributing to errors in result interpretation. In addition, such electrochemical measurement captures overall signal including specific and non-specific interactions, and its implementation could be challenging for low molecular weight targets especially when size of analyte is comparable to that of the redox marker [10].

Thus, the development of other electrochemical approaches that would exclude the utilization of external redox solutions while allowing rapid and sensitive analysis of target molecules by MIP-based sensors would be a great contribution towards a successful diagnostics and environmental remediation.

Ruthenium, Ru is an inert transition metal with a variety of features that have piqued research interest. Its nanoparticles are frequently used in catalytic reactions, water oxidation, pH sensing, and other biological applications [11-14]. Due to their small sample volumes and large dynamic range, Ru NPs are suitable for ultrasensitive detection in biological assays [15]. Moreover, the oxidation states of Ru varies from -2 (in $[Ru(CO)4]^{2-}$) to +8 (in RuO4), thus Ru complexes are redox-active and Ru redox couples are reversible, making them attractive for electrochemical analysis [16].

Additionally, Ru dioxide ($RuO_2$) electrodes have gained prominence in electrochemical applications due to its high thermodynamic and chemical stability that contribute to enhanced performance and versatility, making it a preferred choice in various technological domains. For instance, its mesoporous crystalline structure promotes electrical conductivity by ensuring efficient charge transfer [17]. This enables rapid and sensitive electrochemical measurements that are vital for applications requiring fast response times and high signal-to-noise ratios.

In addition, $RuO_2$ electrodes encompass high stability under harsh experimental conditions, wide potential window that allows the investigation of a broad range of redox reactions, catalytic activity that could be exploited to improve the efficiency of various electrochemical processes, and biocompatibility making it valuable in bioelectrochemical applications where living organisms and biological samples are utilized [18]. Moreover, $RuO_2$ is critical for modifying electrodes that requires high overpotential for generating analyte oxidation current, especially when such overpotential is detrimental to other constituents in the sample media [19].

For example, Pt requires ca. +0.7 V versus Ag/AgCl to generate the oxidation current of $H_2O_2$ but this potential could oxidize ascorbic and uric acids in human blood sample thus interfering with the analyte detection [20]. Economically, Ru, from which $RuO_2$ is obtained, is one of the most affordable platinum group metals electrocatalyst alternatives costing only 4% of Pt. These attributes position the electrode as a pivotal component in the advancement of electrochemical research and applications. Its sensing applications in electrochemistry was lately reviewed by Pitchaimani et al. [18].

Furthermore, $RuO_2$ is applied in supercapacitors chiefly due to its wide range of potential over which reversible redox reactions could take place in addition to its long-life cycle. Hence, several reports exist for different forms of these applications eliciting several recent reviews [17,21,22].

Despite the numerous advantages of $RuO_2$ electrodes, it had not received much attention from the molecular imprinting community, and its consolidation with MIPs to implement real-life analysis of biological or environmental samples is scarcely presented. The primary objective of this invention is application of a BDNF-MIP constructed on a $RuO_2$ electrode as a sensor for the analysis of BDNF in biological samples. The novelty of this solution is in the exclusion of the routine requirement for an external probe solution for the electrochemical measurements.

The synergistic integration of MIPs and $RuO_2$ would not only provides a robust and responsive electrochemical sensing platform, but the internal probe of the electrode presents a versatile platform with applications in diverse fields, including clinical diagnostics, pharmaceutical research, and environmental monitoring.

The developed MIP-based electrochemical sensor is suitable for a detection of electrochemically inactive (or redox-inactive) analytes including a variety of compound groups, such as pharmaceuticals (e.g., antibiotics), pesticides/herbicides as well as peptides and proteins (e.g., viral proteins), preferably neurotrophic factors and more preferably brain-derived neurotrophic factor (BDNF). Electrochemically inactive compound is a compound that remains stable and does not get oxidized or reduced during an electrochemical measurement.

Present invention provides a method for a preparation of a molecularly-imprinted-polymer (MIP) electrochemical sensor for detection of electrochemically inactive analytes. Said sensor comprises at least one electrode (so called electrically conductive surface) with a redox-active surface. Said redox-active surface is made preferably a transition metal oxide, and more preferably ruthenium oxide ($RuO_2$), wherein the redox-active surface is coated by a MIP layer, wherein the MIP layer comprises a plurality of analyte-selective cavities.

In a preferred embodiment of invention, the redox-active surface comprising a ruthenium oxide ($RuO_2$).

The MIP layer can be formed by different synthesis methods, where the formation of a plurality of analyte-selective cavities in a polymer layer can be achieved by various molecular imprinting approaches, including bulk imprinting, surface imprinting and wherein the polymer layer is formed by chemical polymerization, photochemical polymerization and more preferably by electrochemical polymerization.

Present invention provides a method for a preparation of a molecularly-imprinted-polymer (MIP) electrochemical sensor for detection of electrochemically inactive analytes comprising at least one electrode with a redox-active surface.

Said redox-active surface is preferably made of a transition metal oxide, and more preferably ruthenium oxide ($RuO_2$), wherein the redox-active surface is coated by a MIP layer, wherein the MIP layer comprises a plurality of analyte-selective cavities.

According to the invention, the MIP layer is formed according to the method of the invention comprising the steps where:

in step (1) formation of a cleavable linking layer on the electrode,

by a formation of a 3-(2-pyridyldithio) propionyl hydrazide (PDPH) monolayer on the redox-active surface ($RuO_2$) of the electrode; and
by a formation of a 3,3'-dithiobis(sulfosuccinimidyl propionate) (DTSSP) monolayer covalently linked to the 3-(2-pyridyldithio) propionyl hydrazide (PDPH) monolayer;

in step (2) immobilization of an electochemically inactive protein molecules on the cleavable linking layer;
in step (3) polymerization of m-phenylenediamine (m-PD) on the electochemically inactive protein molecules immobilized onto the electrode thereby forming a polymeric layer coating on said electrically conductive surface with entrapped electochemically inactive protein molecules; and
in step (4) cleavage of the cleavable linking layer thereby removing the electochemically inactive protein molecules from the polymeric layer and obtaining the MIP layer.

In the first embodiment of invention, the electochemically inactive protein is preferably a neurotrophic factor, preferably brain-derived neurotrophic factor (BDNF).

The thickness of the MIP layer is adjusted in such a manner that ensuring that the redox-active surface of the electrode, e.g. $RuO_2$, preserves its redox activity in the analyte containing solution within the respective potential window.

The invention also provides a method for using a MIP electrochemical sensor comprising steps, wherein the electrochemical sensor is contacted with a sample solution comprising a target analyte for a defined time period, wherein the electrochemical sensor comprises at least one working electrode, a reference electrode and a counter electrode integrated on the insulative substrate, wherein a working electrode comprises a redox-active surface, wherein the redox-active surface is coated by a molecular imprinted polymer (MIP) layer, and wherein the target analyte selectively binds to a plurality of analyte-selective cavities within the MIP layer. An electrochemical potential is imposed on the working electrode with the aid of a potentiostat. An electrochemical current is measured with the aid of a potentiostat, wherein the electrochemical current is calibrated to a concentration of the target analyte in the sample solution.

In the preferred embodiment of a measuring the electrochemical current comprises measuring the electrochemical current by a voltametric method including, cyclic voltammetry, differential pulse voltammetry and more preferably linear sweep voltammetry, wherein the target analyte binding to the analyte-selective cavities of a MIP layer blocks a redox reaction at the redox-active electrode surface, and wherein the electrochemical current is recorded after an incubation period.

The electrochemical current recorded after an incubation period is normalized to the electrochemical current recorded with the same MIP electrochemical sensor in analyte-free solution.

Present invention also provides a molecularly-imprinted-polymer (MIP) electrochemical sensor for detection of electrochemically inactive analytes, comprising at least one electrode with an electrically conductive surface with a redox-active surface, preferably a transition metal oxide, and more preferably ruthenium oxide ($RuO_2$), wherein the redox-active surface is coated by a MIP layer, wherein the MIP layer comprises a plurality of analyte-selective cavities.

This invention presents an electrochemical sensor based on molecularly imprinted polymer (MIP) that permits direct analysis of analytes without the employment of external probe solution. Said analytes comprising electrochemically inactive analytes, including pharmaceuticals, pesticides, peptides, proteins, antibiotics, neurotrophic factors, e.g., a brain derived neurotrophic factor (BDNF).

By anchoring a MIP to ruthenium dioxide ($RuO_2$) electrode functioning as both a substrate transducer and a redox-active internal probe, a rapid electrochemical analysis of brain derived neurotrophic factor (BDNF), as an exemplary disease biomarker, was achieved.

Following the optimization of the template elution, rebinding time, and MIP layer thickness, linear sweep voltammetry (LSV) analysis of sensor demonstrates low detection limit, for example 0.1 ng/mL in buffer and an effective functionality within two linear analytical ranges (0 - 15 ng/mL and 15 - 40 ng/mL) in serum.

Additionally, exceptional discriminatory selectivity for the target was confirmed in comparison with other neurotrophic factors, as well as other proteins. Importantly, an outstanding reusability of the sensor during at least eight rebinding-regeneration cycles was achieved. Thus, the resulting sensing platform offers rapid and precise analysis of biological samples, paving the way for enhanced diagnostics and analytical methodologies across various scientific disciplines.

In the present invention, in BDNF sensor configuration, the RuO$_2$ electrode serves as both an electrode transducer and a redox probe that can be used as an electrochemical indicator for monitoring target binding on the BDNF-MIP-modified electrode surface.

The recognition principle of BDNF sensor relies on harnessing the strong electrochemical redox properties of RuO$_2$ to monitor changes in selective analyte adsorption at proximity of the electrode surface. Thus, during LSV, the RuO$_2$ electrode undergoes oxidation, generating charge carriers that transfer between electrode and buffer solution through the empty binding cavities of the BDNF-MIP layer, thereby sustaining the current in the electrochemical cell.

If the buffer solution contains BDNF, the protein rebinds on the binding cavities on the BDNF-MIP, and as a result a concentration-dependent obstruction of the charge transfer occurs, leading to a noticeable change in the measured current. In this configuration, the sensor signal measurement is conducted directly in the analyte solution, eliminating two additional steps required in the previous sensor design, like described in prior art US11193940B2.

As a result, the measurement process becomes simpler and faster. Since RuO$_2$ is covered by a MIP layer, its contact with a sample is minimized preserving RuO$_2$ redox activity for longer usage/reusage period.

Additionally, this arrangement prevents the analyte from contacting the redox probe, which helps avoid potential interactions or influences on the analyte molecule, such as changes in protein conformation.

The other differences compared to the closest prior art US11193940B2 are:

- use of 3-(2-pyridyldithio) propionyl hydrazide (PDPH) linker on RuO$_2$ instead of 4-ATP on Au;

- use of linear sweep voltammetry (LSW) instead of differential pulse voltammetry (DPV).

BRIEF DESCRIPTION OF DRAWINGS

Present invention is described below with reference to the accompanying schematic drawings, in which:

Figure 1 is a scheme illustrating the operating principle of the sensor employing a MIP layer on RuO$_2$.

Figure 2 illustrates an optimization of sensor preparation.

Figure 3 depicts a calibration plot of BDNF sensor obtained within the concentration range of 0.1 - 1.0 ng/mL) in PBS.

Figure 4 illustrates different sensor responses.

Figure 5 illustrates an electivity of BDNF sensor.

Figure 6 illustrates responses of BDNF-MIP and NIP based sensors.

DESCRIPTION OF EMBODIMENTS

**2. Experimental**

**2.1. Chemicals and Materials**

Dithiothreitol (DTT), m-phenylenediamine (mPD), sodium chloride (NaCl) and acetic acid (AcOH) 99.8% were obtained from Sigma-Aldrich. 3,3'-dithiobis (sulfosuccinimidyl propionate) (DTSSP) and 3-(2-pyridyldithio) propionyl hydrazide (PDPH) were obtained from Thermo Fisher Scientific Inc. Human recombinants of BDNF (27 kDa, pI 9.43), mesencephalic astrocyte-derived neurotrophic factor, MANF (18.1 kDa, pI 8.55), cerebral dopamine neurotrophic factor, CDNF (18.5 kDa, pI 7.68), and cluster of differentiation 48, CD48 antigen (22.2 kDa, pI 9.36), SARS-Cov-2 nucleoprotein ncovNP (45 kDa, pI 10.07) were supplied by Icosagen AS (Tartu, Estonia). E2 (23 kDa, pI 8.24) and CD81 (20 kDa, pI 5.40) were purchased from ServiceGen (St. Petersburg, Russia). Prostate-specific antigen, PSA (26 kDa, pI 7.26) was obtained from Temecula (California, USA). Sodium phosphate dibasic dodecahydrate, potassium dihydrogen phosphate, and potassium chloride were from Lach-ner. Fetal bovine serum (FBS) was obtained from Pan Biotech (Aidenbach, Germany). All aqueous solutions were prepared using ultrapure water (with a resistivity of 18.2 MQ.cm, Merck KGaA, Darmstadt, Germany).

Analyte solutions were prepared using PBS with a molar concentration of 10 mM and a pH value of 7.4. The Drop Sense RuO$_2$ electrodes (DRP-810) were obtained from Metrohm (Metrohm DropSens, Spain). DRP-810 consist of a working electrode (WE) (4 mm diameter) made of ruthenium oxide, a reference electrode (RE) made of silver, and an auxiliary electrode (AE) made of carbon.

## 2.2. Modification of RuO$_2$ electrode with BDNF-MIP layer

The BDNF-MIP modified RuO$_2$ electrode was created by coating WE of DRP-810 with BDNF-MIP film made from poly-m-phenylenediamine (PmPD). A computational modelling technique was used to rationally identify mPD as a suitable functional monomer [23]. The WE of the DRP-810 was modified with PDPH by incubating it in a 1 mM PDPH solution in phosphate buffered saline (PBS) for 30 min and then washing with PBS. To create the cleavable linker monolayer, 100 µL (10 mM) DTSSP solution was dropped onto PDPH/RuO$_2$ and rinsed with PBS after 30 min. To immobilise BDNF, 10 µL of PBS with 0.025 mg/mL of BDNF was dropped on the cleavable linker modified electrode for 30 min and rinsed well.

Molecular imprints of BDNF in the polymer film were created by treating the polymer film with 50 mM DTT in PBS to cleave the S-S link of DTSSP and promote BDNF release, then washing with a 10% acetic acid solution. A similar process was used for the reference film, non-imprinted polymers (NIPs), with the exception of the BDNF immobilisation phase, to prevent the appearance of BDNF imprints in this film. The sensor preparation stages were characterised by cyclic voltammetry (CV) in 10 mM PBS solution at a potential range of - 0.4 to 0.6 with a scan rate of 50 mV/s vs Ag/AgCl/KClsat as the reference electrode.

## 2.3. Rebinding and selectivity studies

The sensor's capacity to recognise the target analyte was determined using linear sweep voltammetry (LSV) in a PBS-based analyte solution, which utilised internal ruthenium oxide redox probe characteristics. The LSV was conducted in the potential range of -0.2-0.3V vs external Ag/AgCl/KClsat reference, with a scan rate of 50 mV and a step size of 0.5 mV. To conduct the rebinding investigation, sensors were incubated in PBS containing increasing analyte concentrations (1-100 ng/mL), followed by LSV measurements. The anodic current peaks corresponding to each analyte concentration were normalised to the base current at the zero-analyte concentration using Eq. (1):

$$I_n = (I_0 - I)/I_0 \qquad\qquad (1)$$

where $I_0$ and $I$ are the LSV current peaks measured after incubation in PBS buffer solutions without and with analyte, respectively.

The responses of BDNF sensor and its reference (sensor with NIP-modified electrode) to increasing analyte concentrations were plotted as binding isotherms and fitted to the Langmuir adsorption model. This analysis was then used to assess the effect of different sensor modifications yielding the dissociation constant (KD) and maximum binding response at saturation ($I_{n,sat}$) (Eq. 2).

$$I_n = (I_{n,sat}C)/(K_D + C) \qquad\qquad (2)$$

where $I_n$ is responses at concentration C. The capability of the BDNF sensor to recognize the target protein was estimated by imprinting factor (IF) using Eq. 3:

$$IF = I_{n,sat(MIP)}/I_{n,sat(NIP)} \qquad\qquad (3)$$

where $I_{n,sat(MIP)}$ and $I_{n,sat(NIP)}$ are saturated responses of BDNF-MIP- and NIP-modified electrodes, respectively, against BDNF.

Additional experimental tests with fetal bovine serum (FBS) samples were conducted to assess the practicality of employing the sensor to measure BDNF in complicated samples as well as sensor selectivity. The samples were generated by spiking a suitable amount of BDNF or interfering protein into an empirically determined optimal FBS dilution. The sensor's response to each diluted sample was tested after initial incubation.

## 3. Results and discussion

### 3.1. Preparation of BDNF sensor

The surface imprinting strategy employed in the sensor preparation involves careful growth of a polymer film around a protein template anchored on an electrode surface. To anchor BDNF on the electrode, we employed the pyridyl dithiol functional group of PDPH to form stable complex with Ru atom using its coordination bonds between nitrogen and sulphur atoms. PDPH linker consists sterically accessible amino groups that do not participate in RuO$_2$-PDPH complex formation thus resulting in the amination of the electrode surface [24,25]. These free amino group form covalent bonds with one symmetric group of the homo-bifunctional cleavable linkers, DTSSP, that can bind a lysine residue of BDNF molecule.

After cementing the protein within the polymer, its subsequent removal is necessary to reveal the binding cavities and obtain the BDNF-imprinted polymer (BDNF-MIP). This was achieved by a dual process involving an initial cleavage of the disulphide bridge of DTSSP linking the protein to the electrode surface by DTT followed by protein removal by treatment in AcOH solution.

Each stage of BDNF sensor preparation protocol was closely monitored by CV, where the modification of electrode by the non-conducting layer was expected to induce a corresponding change in capability for charge transfer through the electrode surface. Clearly, increasing reduction in the anodic and cathodic currents in CV was observed following step by step electrode modifications with PDPH, DTSSP, BDNF and PmPD, indicating surface passivation by the electrochemically inactive materials. However, upon treatment with DTT and AcOH and copious washing with PBS, significant amounts of the hitherto disappeared peaks were restored.

This indicates the effectiveness of the cleavage and washing out procedures that allow for removal of the trapped template molecules thereby creating molecular cavities in the polymer through which improved charge transfer was enabled.

### 3.2. Sensor operating principle

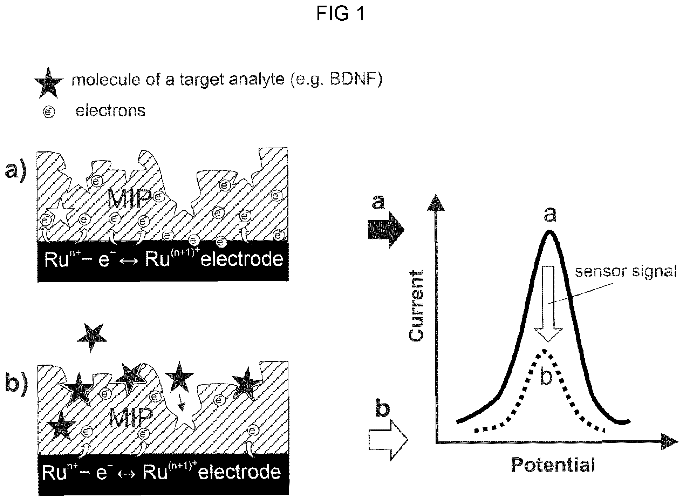

The recognition principle of BDNF sensor relies on harnessing the strong electrochemical redox properties of $RuO_2$ to monitor changes in selective analyte adsorption at proximity of the electrode surface. $RuO_2$ is a multivalent oxide having multiple redox states within a certain range of potential [26]. Thus, in BDNF sensor configuration, the $RuO_2$ electrode serves as a redox centre that can be used as an electrochemical indicator for monitoring target binding on the BDNF-MIP-modified electrode surface. During LSV the $RuO_2$ electrode is oxidised and provides the charge carriers supporting the current in the electrochemical cell through the binding cavities of BDNF-MIP thin film layer and the diluted serum-containing buffer solution, serving as an electrolyte. After sensor incubation in buffer solution containing BDNF, the latter rebinds on the preformed cavities on the BDNF-MIP, a concentration-dependent obstruction of the charge transfer occurs, leading to a noticeable change in the measured currents (Figure 1).

Figure 1 contains a scheme of the operating principle of the BDNF sensor. In the absence of BDNF, charge is transferred from the $RuO_2$ electrode surface through the binding cavities on BDNF-MIP thin film into the buffer solution of the diluted serum, providing strong current peak (green line). After BDNF rebinding, the non-conducting protein molecules obstruct the charge transfer, resulting in a reduced current (red line).

### 3.3. Sensor Optimization and performance

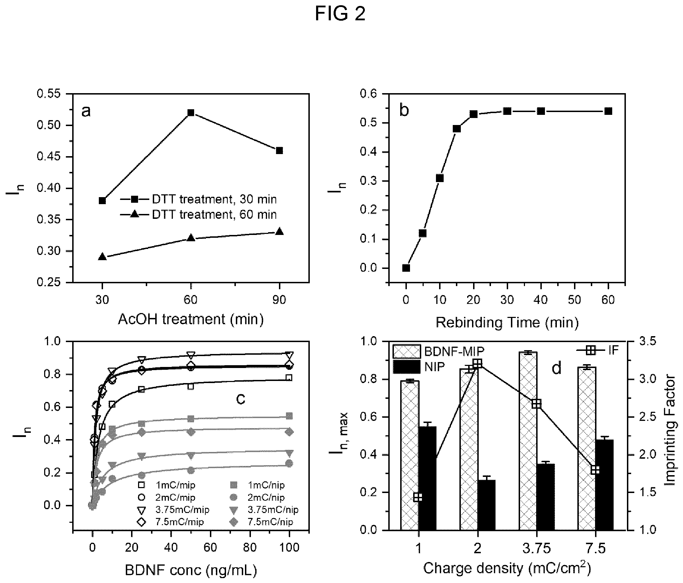

The optimization of a MIP-based sensor is essential to achieving its excellent performance and a reliable and reproducible analytical result. Typically, this entails adjusting several parameters in its preparation and validation steps, thereby enhancing the sensor's overall performance. In this work, we focused on choosing the optimal timing for disulfide bridge cleavage, template elution, and target rebinding, in addition to optimizing the electrochemical charge density at which the polymer is electrodeposited on the electrode. Figure 2 shows the results obtained for the different optimizations.

The performance of a MIP-based sensor is strongly dependent on the availability of target-specific cavities created within the polymer. Thus, the efficiency of template removal constitutes a critical step to the overall effectiveness of a MIP-based sensing device. As seen in Fig. 2a, cleavage of the disulfide bridge by treating the BDNF-entrapped polymer modified electrode with DTT for 30 min, clearly resulted in the more responsive BDNF sensor (as demonstrated by higher In values) than the 60 min treatment. This indicates a more efficient template release in a shorter time frame. Further incubation in AcOH for 60 min produced the highest sensor response due to the increased efficacy of template elution. Thus, treating the produced polymer with DTT and AcOH for 30 and 60 min, respectively, effectively removes the template and reveals the target-specific polymer cavities.

Figure 2 illustrates an optimization of sensor preparation. Sensor's response to 1 ng/mL concentration of BDNF at different (a) combinations of cleavage/washing out and (b) rebinding times. In Figure 2, (c) illustrates adsorption isotherms of BDNF-MIP and NIP-modified sensors prepared at different charge densities (ca. 1, 2, 3.75, 7.5 $mC/cm^2$) for increasing analyte concentration (0-100 ng/mL). Bold lines represent Langmuir fits to the experimental data to derive (d) saturated responses (In,sat) of BDNF-MIP and NIP-modified sensor and imprinting factor (IF) values obtained at the different charge densities.

Moreso, the optimal time required to rebind the target on the sensor was determined by measuring the response induced by same concentration of BDNF (1 ng/mL) on the sensor after incubating in analyte solution for varying times (5 - 60 min), in Figure 2, (b).

The analysis reveals that the reaction grows steadily with increasing incubation time until 15 min, after which it increases considerably more slowly until 30 min. After this time, a slight decline could be observed, suggesting that 30 min incubation time is optimal for efficient rebinding of the target molecules to the pre-formed functional units in the binding cavities. Finally, attention was shifted to the effect of polymer thickness on the sensor performance. Our research group has earlier demonstrated for several target molecules that the same polymer, but with different thicknesses, exhibits varying rebinding properties [27-29].

Thus, it is essential to determine which polymer thickness engender the best performance of the MIP-based sensor e.g. by preventing irreversible entrapment of the template proteins and ensuring adequate charge transfer to the electrode surface [23]. Consequently, different densities of the polymer layer were formed on an individual electrode by varying the charge densities (1, 2, 4 and 8 $mC/cm^2$) employed in the electrodeposition. To eliminate contribution to the response from nonspecific interactions, reference polymer layers, termed non-imprinted polymers (NIPs), were prepared using the same protocol but excluding template washing stages. After this, the responses of the respectively modified sensors to increasing concentration of the analyte were measured and plotted as adsorption isotherms (Figure 2, (c)). As observed, the Langmuir model accurately fits the experimental data and clearly indicates higher induced responses on the electrodes modified by the BDNF-MIPs than those on the NIPs, at the varying charge densities. This reflects the importance of creating target-selective cavities within the polymer.

Furthermore, in Figure 2, (d) shows that responses on BDNF-MIP increase with charge density from 1 to 3.75 $mC/cm^2$ and then decline afterwards, while those on NIP decrease sharply from 1 to 2 $mC/cm^2$ followed by a steady increase beginning at 3.75 $mC/cm^2$. The relative adsorption as calculated by the imprinting factor, IF (Eq. 3), demonstrates that a high specific response is obtainable from BDNF-MIP prepared from 2 $mC/cm^2$ and it is accompanied by a low response from non-specific interactions, as identified on the NIP, yielding an IF value of 3.2 (See Table S1). Hence, an ideal sensor performance could be obtained from a sensor constructed at 2 $mC/cm^2$, indicating the requirement of a MIP layer that is just thin enough to preclude major non-specific interactions. Thus, 2 $mC/cm^2$ was selected as the optimal electrodeposition charge density for producing BDNF-MIP film with the best analytical performance on the electrode.

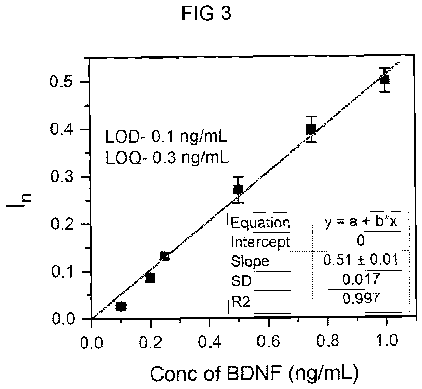

Following optimization in the buffer, the sensor was tested against low analyte concentrations to determine its analytical range such as detection (LOD) and quantification (LOQ) limits. The induced responses were plotted against each concentration and a linear regression of the data was obtained (Figure 3).

By inputting the parameters obtained from the linear regression (inset to Fig. 3) into Eqs. 4 and 5, an LOD of 0.1 ng/mL and LOQ corresponding to 0.3 ng/mL were obtained:

$$LOD = 3*SD/b \qquad\qquad (4)$$

$$LOQ = 10*SD/b \qquad\qquad (5)$$

where, respectively, $SD$ and $b$ stand for the standard deviation and the slope of regression line.

Although the limits are higher than what was obtained in our previous report [23], the sensor still demonstrates capacity for BDNF analysis since in normal serum the target exists within a concentration range of 8 and 40 ng/mL [30-33].

**Real sample analysis**

Clinical diagnosis involves the analysis of biological samples, mainly serum, plasma, urine, or tissue extracts, with varying degrees of complexity. Thus, the evaluation of MIP-based sensors against any of these samples could represent a realistic validation of their performance and could help in determining their practical utility in clinical settings. However, BDNF varies from person to person, hence a random collection of clinical samples for BDNF analysis could be challenging. In this work, the clinical practicability of the sensor was examined by analysing its performance in fetal bovine serum (FBS) samples. FBS is considered a useful substitute to human serum in the analysis of BDNF in biological samples because of its possession of negligible amount of endogenous BDNF, while being similar to human serum in other major constituents [34].

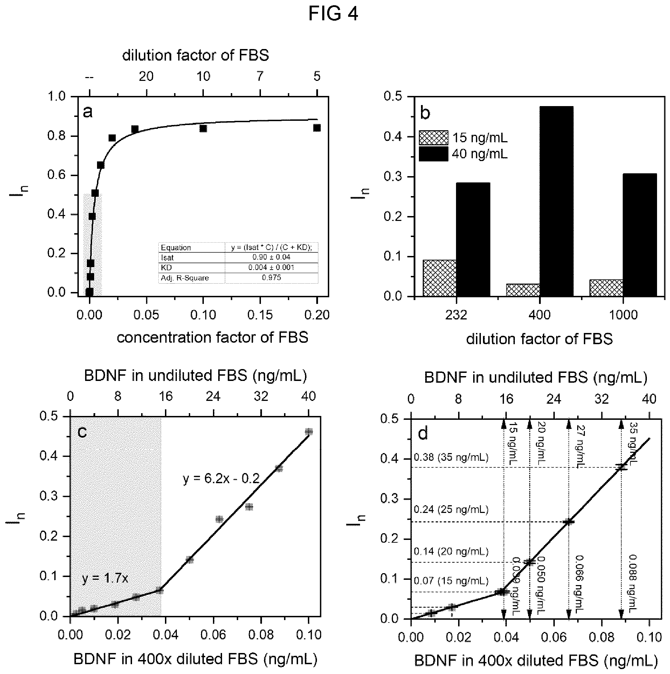

In Figure 4, (a) illustrates BDNF sensor responses measured at different FBS dilutions. Bold line represents Langmuir's fit to the experimental data.

In Figure 4, (b) illustrates the responses of the sensor to two BDNF concentrations (simulating 15 and 40 ng/mL in undiluted FSB) spiked in different FBS dilutions viz: 232-, 400- and 1000-fold dilutions.

In Figure 4, (c) illustrates the linear calibration plot determined from the responses of BDNF sensors induced by different BDNF concentrations in 400-fold diluted FBS.

In Figure 4, (d) illustrates the correlation between spiked concentrations of BDNF, black dots, and the sensor-determined concentrations, green dots, for each blind sample. An incubation time of 30 min was used and at least 3 repetitive measurements were performed for each sample.

However, prior to measurements in FBS an initial optimization of FBS serial dilution was conducted, as a customary practice in many biological sample preparation protocols. This helps to avoid signal saturation and eliminates interferences from other compounds in the sample that could falsely affect the results, thereby ensuring reproducibility and the quality of the data collected [35]. By analysing the sensor's response against FBS dilution, we aimed to determine conditions where the sensor would exhibit increased sensitivity to the target protein injection. The most sensitive range was evidently below 50% of the sensor's maximum response (Figure 4, (a)).

Fitting the data to the Langmuir model yielded KD values corresponding to a 232-fold dilution, representing 50% of the sensor's maximum response. Upon further experimental verification with two BDNF spiked in concentrations (Figure 4, (b)), it was evident that 400-fold dilution demonstrated even better suitability for the analysis by permitting a higher analyte-induced response. Hence, 400-fold FBS dilution was selected as optimal dilution for further analysis.

To better evaluate the sensor performance in optimally diluted FBS, it was spiked with known concentrations of BDNF to determine its linear variation with increasing analyte concentrations, starting from 15 ng/mL. The result show that a linear relationship, up to 40 ng/mL could be established (Figure 4, (c)). However, upon further analysis below 15 ng/mL, another linear variation, howbeit with a different slope, was observed (Fig. 4c). The emergence of two linear calibration plots having different slopes likely indicates two separate binding sites possessing distinct affinities [36].

Following this, blind samples containing spiked-in BDNF in FBS were measured and their induced responses determined. This is to validate the analytical feasibility of the sensor in determining unknown concentrations of BDNF in clinical sample. BDNF concentrations were estimated by extrapolation from the calibration plot (Figure 4, (c)), using the obtained responses, and then compared to the actual BDNF amount in each sample. Figure 4d indicates the existence of a near perfect correlation between the sensor determined concentration and the actual spiked-in BDNF amounts with percentage recoveries ranging from 92 to 170% (Table 1). The outcome thus points to the sensor's ability to distinguish between serum samples with varying concentrations of BDNF.

Table 1. The correlation between spiked and measured concentrations of BDNF from blind FBS samples and the resulting recoveries.

| Spiked conc. (ng/mL) | Measured conc. (ng/mL) | Recovery (%) |
|---|---|---|
| $2.0 \pm 0.1$ | $3.4 \pm 0.1$ | $170 \pm 20$ |
| $7.5 \pm 0.2$ | $7.0 \pm 0.3$ | $92 \pm 5$ |
| $15 \pm 1$ | $15 \pm 1$ | $101 \pm 10$ |
| $20 \pm 2$ | $20 \pm 3$ | $100 \pm 20$ |
| $25 \pm 1$ | $27 \pm 4$ | $106 \pm 16$ |
| $35 \pm 3$ | $35 \pm 3$ | $101 \pm 12$ |

**Selectivity**

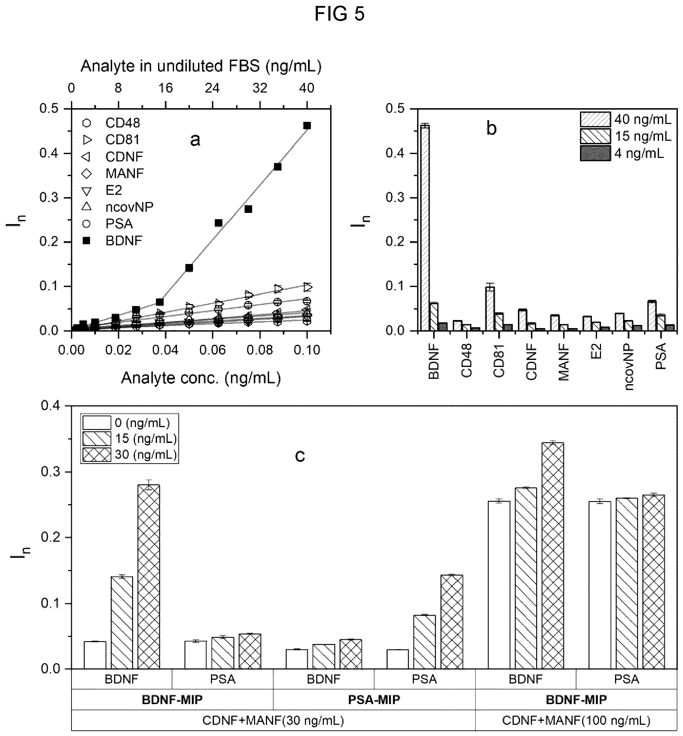

Selectivity was assessed by testing the sensor against interfering biomolecules that could present themselves in the clinical samples of neurodegenerative patients as well as other biomolecules that could accompany or premeditate such disorder. Hence, 400-fold diluted FBS was spiked with required amount of CD48, CDNF, CD81, MANF, E2, ncovNP or PSA and their induced response on the sensor was compared to those of BDNF. As seen in Figure 5, (a), BDNF induced a significantly higher responses on the sensor than other tested interferents within the same concentration range. In fact, other neurotrophic factor proteins including MANF and CDNF are similar with BDNF in molecular weight and isoelectric points, but different in functional values and sequence, show very low responses to similar concentrations (Figure 5, (b)), indicating the specificity in the nature of the preformed recognition units in the binding cavities on the polymer.

Figure 5 illustrates a selectivity of BDNF sensor, wherein responses of BDNF sensor to different interfering molecules in 400-fold diluted FBS samples (a) at a concentration range between 0 and 0.1 ng/mL corresponding to a simulated (undiluted) concentration between 0 and 40 ng/mL, (b) at three different concentrations 0.01, 0.0375, and 0.1 ng/mL (i.e., 4, 15 and 40 ng/mL). (c) Responses of BDNF-MIP and PSA-MIP based sensors to 0.0375 and 0.075 ng/mL (i.e. simulated concentrations of 15 and 30 ng/mL respectively) of BDNF and PSA in 400-fold diluted FBS containing 0.075 ng/mL and 0.25 ng/mL (i.e. simulated concentrations of 30 ng/mL and 100 ng/mL) each of both CDNF and MANF. Lines represent linear fits to the experimental data. All data points are mean of three independent measurements from three sensors.

Moreover, to evaluate the sensor's selectivity further, competitive assays were conducted where two neurotrophic factor proteins (CDNF or MANF) competed with BDNF for occupancy of the binding sites on BDNF-MIP (Figure 5 (c)). Herein, the concentrations of both interfering proteins were fixed at 0.075 ng/mL, corresponding to a simulated concentration of 30 ng/mL. Clearly, BDNF induced a notably higher response on the sensor at both simulated tested concentrations of 15 and 30 ng/mL than PSA despite the presence of both interfering compounds at a similar concentration level. This result established the discriminatory selectivity of the sensor for the target. Additionally, to further demonstrate the adaptability of the selectivity, PSA-MIP was created using the same method as BDNF-MIP, but with PSA serving as the imprinted template molecule. Subsequently, a comparable experiment was carried out on the PSA-MIP-based sensor (Figure 5 (c)).

The analysis reveals that BDNF only succeeded in eliciting a modest response, which does not rise appreciably when the concentration of BDNF was increased from 15 to 30 ng/mL. Finally, BDNF sensor's discriminating abilities against higher concentrations of CDNF and MANF (i.e. 0.25 ng/mL which correspond to a simulated amount of 100 ng/mL) were examined (Figure 5 (c)). Apparently, even at such high doses of interferents, lower concentration of BDNF caused a discernible reaction. Moreso, at both tested concentrations (0.0375 and 0.075 ng/mL), the sensor showed a noticeably stronger recognition for BNDF than PSA.

Thus, the practical viability of using the sensor for clinical assessment of complex clinical samples where the interferents could exist at quantities different from the target, is further supported by this result. The findings unmistakably demonstrate the target-selective nature of the BDNF sensor and highlight its enormous potential for use as a trustworthy analytical tool for the clinical assessment of disease biomarker in biological samples.

**Regeneration**

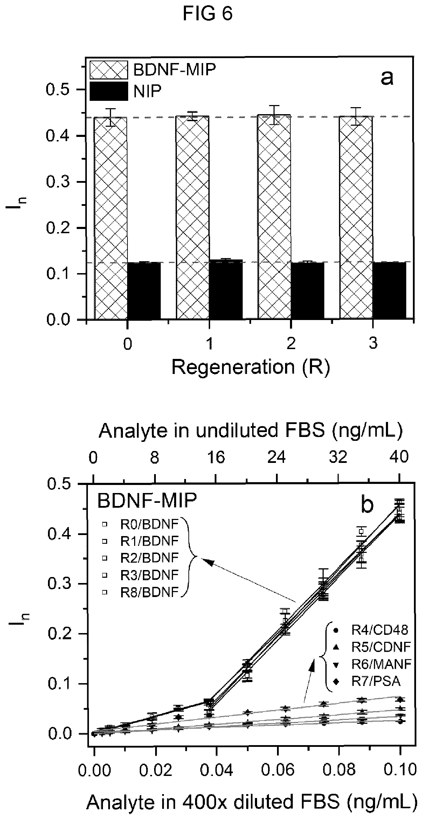

A vital requirement of an analytical sensor is the possibility to be re-used. Although, MIPbased sensors are generally considered a cheaper alternative to bio-based recognition units, the ability to re-use them after a rebinding and regeneration cycle constitutes an added advantage. Thus, to examine the sensor reusability, the sensor was subjected to a series of regeneration-rebinding cycles. The regeneration was achieved by subsequent washing out processes leading to recovery of the binding cavities. Fig. 6a shows BDNF-MIP and NIP responses to 0.1 ng/mL concentration of BDNF after three regeneration-rebinding cycles.

As observed, BDNF-MIP indicates no noticeable change in the analyte-induced responses over the three cycles and the responses are comparable to the response on the freshly prepared surface (R0). The reference NIP also shows similar stable response for the three cycles. Certainly, the result confirms the stability of the polymer-$RuO_2$ system and thus, strengthens the practical adoption of the sensor as a commercially viable alternative analytical system for clinical analysis of disease biomarkers in a reproducible and reusable manner.

Furthermore, to study the effectiveness of the recognition units within the binding cavities following prolonged regeneration-rebinding cycles, more regeneration and rebinding were performed howbeit with interferents. Thus, CD48, CDNF, MANF, and PSA were tested following regeneration 4, 5, 6, and 7 (R4, R5, R6, and R7), respectively.

Expectedly, the responses obtained as seen in Figure 6 (b) remained low over the entire concentration range. However, upon further regeneration and rebinding of BDNF within the same range of concentration, a rapid and superior response (R8) that is like those of R0-R3, is obtained. Convincingly, the sensor demonstrates a strong affinity in the recognition of the target molecules whether freshly prepared, after several repeated usage or after prior exposure to other protein molecules at similar or higher concentrations.

In Figure 6, (a) illustrates responses of BDNF-MIP and NIP based sensors to 0.1 ng/mL concentrations (i.e. 40 ng/mL simulated concentration) of BDNF in 400-folds diluted FBS following increasing number of regeneration-rebinding cycle (b) responses of BDNF sensor to increasing simulated concentration (0 - 40 ng/mL) of BDNF initially after three regeneration-rebinding cycle (R0 - R3), followed by those of interferents (R4 - R7) and finally BDNF (R8). Lines represent linear fit to the experimental data. All data points are mean of three independent measurements from three sensors.

**Conclusion**

This invention provides an electrochemical sensor in which a molecularly imprinted polymer (MIP) with selective molecular cavities for BDNF is tethered to a ruthenium IV oxide ($RuO_2$)-based electrode that serves as both a transducer and an internal redox probe. By combining $RuO_2$'s strong redox capabilities with a MIP layer, a simple electrochemical system is created that eliminates the need for an external probe solution while yet allowing for rapid and high-performance measurement of target analytes.

Following optimisation, the sensor exhibits remarkable sensitivity to the target, as evidenced by a LOD of 0.1 ng/mL in PBS and a detectable simulated concentration of 1 ng/mL in serum. Its practical usefulness for analysis in complex media was explored and confirmed in fetal bovine serum, which showed high selectivity as seen by robust discrimination against even closely related neurotrophic factor proteins. Furthermore, the sensor demonstrated excellent repeatability and stability, withstanding up to 8 rebinding-regeneration cycles without losing significant features.

Although other solutions incorporate redox probes into the MIP layer, in this case, $RuO_2$ is covered by a MIP layer, minimising $RuO_2$ contact with a sample and preventing potential interference with the recognition units of the binding cavities, thereby preserving its activity for longer usage/reusage. This novel cutting-edge analytical approach can be applied to in-vitro medical diagnostics or field environmental monitoring, where rapid and simple detection of target substances in liquid samples is required.

REFERENCES

[1] S. Singh, V. Kumar, D.S. Dhanjal, S. Datta, R. Prasad, J. Singh, Biological Biosensors for Monitoring and Diagnosis, in: J. Singh, A. Vyas, S. Wang, R. Prasad (Eds.), Microb. Biotechnol. Basic Res. Appl., Springer Singapore, Singapore, 2020: pp. 317-335. https://doi.org/10.1007/978-981-15-2817-0_14.

[2] A.G. Ayankojo, J. Reut, V. Syritski, Electrochemically Synthesized MIP Sensors: Applications in Healthcare Diagnostics, Biosensors 14 (2024) 71. https://doi.org/10.3390/bios 14020071.

[3] A.G. Ayankojo, J. Reut, V.B.C. Nguyen, R. Boroznjak, V. Syritski, Advances in Detection of Antibiotic Pollutants in Aqueous Media Using Molecular Imprinting Technique-A Review, Biosensors 12 (2022) 441. https://doi.org/10.3390/bios12070441.

[4] J.J. BelBruno, Molecularly Imprinted Polymers, Chem. Rev. 119 (2019) 94-119. https://doi.org/10.1021/acs.chemrev.8b00171.

[5] D. Elfadil, A. Lamaoui, F. Della Pelle, A. Amine, D. Compagnone, Molecularly Imprinted Polymers Combined with Electrochemical Sensors for Food Contaminants Analysis, Molecules 26 (2021) 4607. https://doi.org/10.3390/molecules26154607.

[6] P.S. Sharma, A. Garcia-Cruz, M. Cieplak, K.R. Noworyta, W. Kutner, 'Gate effect' in molecularly imprinted polymers: the current state of understanding, Curr. Opin. Electrochem. 16 (2019) 50-56. https://doi.org/10.1016/j.coelec.2019.04.020.

[7] A.G. Ayankojo, J. Reut, V. Ciocan, A. Öpik, V. Syritski, Molecularly imprinted polymer-based sensor for electrochemical detection of erythromycin, Talanta 209 (2020) 120502. https://doi.org/10.1016/j.talanta.2019.120502.

[8] A.G. Ayankojo, R. Boroznjak, J. Reut, A. Öpik, V. Syritski, Molecularly imprinted polymer based electrochemical sensor for quantitative detection of SARS-CoV-2 spike protein, Sens. Actuators B Chem. 353 (2022) 131160. https://doi.org/10.1016/j.snb.2021.131160.

[9] P. Lach, M. Cieplak, K.R. Noworyta, P. Pieta, W. Lisowski, J. Kalecki, R. Chitta, F. D'Souza, W. Kutner, P.S. Sharma, Self-reporting molecularly imprinted polymer with the covalently immobilized ferrocene redox probe for selective electrochemical sensing of p-synephrine, Sens. Actuators B Chem. 344 (2021) 130276. https://doi.org/10.1016/j.snb.2021.130276.

[10] A. Yarman, F.W. Scheller, How Reliable Is the Electrochemical Readout of MIP Sensors?, Sensors 20 (2020) 2677. https://doi.org/10.3390/s20092677.

[11] Z. Shi, J. Li, Y. Wang, S. Liu, J. Zhu, J. Yang, X. Wang, J. Ni, Z. Jiang, L. Zhang, Y. Wang, C. Liu, W. Xing, J. Ge, Customized reaction route for ruthenium oxide towards stabilized water oxidation in high-performance PEM electrolyzers, Nat. Commun. 14 (2023) 843. https://doi.org/10.1038/s41467-023-36380-9.

[12] P.K. Gupta, L. Mishra, Ecofriendly ruthenium-containing nanomaterials: synthesis, characterization, electrochemistry, bioactivity and catalysis, Nanoscale Adv. 2 (2020) 1774-1791. https://doi.org/10.1039/D0NA00051E.

[13] M.R. Axet, K. Philippot, Catalysis with Colloidal Ruthenium Nanoparticles, Chem. Rev. 120 (2020) 1085-1145. https://doi.org/10.1021/acs.chemrev.9b00434.

[14] R.H.G. Mingels, S. Kalsi, Y. Cheong, H. Morgan, Iridium and Ruthenium oxide miniature pH sensors: Long-term performance, Sens. Actuators B Chem. 297 (2019) 126779. https://doi.org/10.1016/j.snb.2019.126779.

[15] Singha, P. P., Ruthenium compounds: a new approach in nanochemistry, in: Ruthenium Chem., Pan Stanford Publishing, 2018: pp. 91-119.

[16] Mishra, A. K., & Mishra, L., Ruthenium chemistry, Pan Stanford Publishing, 2018.

[17] D. Majumdar, T. Maiyalagan, Z. Jiang, Recent Progress in Ruthenium Oxide-Based Composites for Supercapacitor Applications, ChemElectroChem 6 (2019) 4343-4372. https://doi.org/10.1002/celc.201900668.

[18] P. Veerakumar, S.-T. Hung, P.-Q. Hung, K.-C. Lin, Review of the Design of Ruthenium-Based Nanomaterials and Their Sensing Applications in Electrochemistry, J. Agric. Food Chem. 70 (2022) 8523-8550. https://doi.org/10.1021/acs.jafc.2c01856.

[19] C.-C. Kung, P.-Y. Lin, F.J. Buse, Y. Xue, X. Yu, L. Dai, C.-C. Liu, Preparation and characterization of three dimensional graphene foam supported platinum-ruthenium bimetallic nanocatalysts for hydrogen peroxide based electrochemical biosensors, Biosens. Bioelectron. 52 (2014) 1-7. https://doi.org/10.1016/j.bios.2013.08.025.

[20] K.-J. Chen, K. Chandrasekara Pillai, J. Rick, C.-J. Pan, S.-H. Wang, C.-C. Liu, B.-J. Hwang, Bimetallic PtM (M=Pd, Ir) nanoparticle decorated multi-walled carbon nanotube enzyme-free, mediator-less amperometric sensor for H2O2, Biosens. Bioelectron. 33 (2012) 120-127. https://doi.org/10.1016/j.bios.2011.12.037.

[21] S.A. Delbari, L.S. Ghadimi, R. Hadi, S. Farhoudian, M. Nedaei, A. Babapoor, A. Sabahi Namini, Q.V. Le, M. Shokouhimehr, M. Shahedi Asl, M. Mohammadi, Transition metal oxide-based electrode materials for flexible supercapacitors: A review, J. Alloys Compd. 857 (2021) 158281. https://doi.org/10.1016/j.jallcom.2020.158281.

[22] R. Liang, Y. Du, P. Xiao, J. Cheng, S. Yuan, Y. Chen, J. Yuan, J. Chen, Transition Metal Oxide Electrode Materials for Supercapacitors: A Review of Recent Developments, Nanomaterials 11 (2021) 1248. https://doi.org/10.3390/nano11051248.

[23] A.G. Ayankojo, R. Boroznjak, J. Reut, J. Tuvikene, T. Timmusk, V. Syritski, Electrochemical sensor based on molecularly imprinted polymer for rapid quantitative detection of brain-derived neurotrophic factor, Sens. Actuators B Chem. 397 (2023) 134656. https://doi.org/10.1016/j.snb.2023.134656.

[24] Y. Peng, G. Yi, Z. Gao, A highly sensitive microRNA biosensor based on ruthenium oxide nanoparticle-initiated polymerization of aniline, Chem. Commun. 46 (2010) 9131. https://doi.org/10.1039/c0cc01990a.

[25] Y. Peng, Z. Gao, Amplified Detection of MicroRNA Based on Ruthenium Oxide Nanoparticle-Initiated Deposition of an Insulating Film, Anal. Chem. 83 (2011) 820-827. https://doi.org/10.1021/ac102370s.

[26] B. Chakraborty, Electrochemical Properties of Sputtered Ruthenium Oxide Neural Stimulation and Recording Electrodes, Electrochem 4 (2023) 350-364. https://doi.org/10.3390/electrochem4030023.

[27] V.B.C. Nguyen, A.G. Ayankojo, J. Reut, J. Rappich, A. Furchner, K. Hinrichs, V. Syritski, Molecularly imprinted co-polymer for class-selective electrochemical detection of macrolide antibiotics in aqueous media, Sens. Actuators B Chem. 374 (2023) 132768. https://doi.org/10.1016/j.snb.2022.132768.

[28] M. Antipchik, J. Reut, A.G. Ayankojo, A. Öpik, V. Syritski, MIP-based electrochemical sensor for direct detection of hepatitis C virus via E2 envelope protein, Talanta 250 (2022) 123737. https://doi.org/10.1016/j.talanta.2022.123737.

[29] A.G. Ayankojo, J. Reut, A. Öpik, V. Syritski, Sulfamethizole-imprinted polymer on screen-printed electrodes: Towards the design of a portable environmental sensor, Sens. Actuators B Chem. 320 (2020) 128600. https://doi.org/10.1016/j.snb.2020.128600.

[30] Y. Naegelin, H. Dingsdale, K. Säuberli, S. Schädelin, L. Kappos, Y.-A. Barde, Measuring and Validating the Levels of Brain-Derived Neurotrophic Factor in Human Serum, Eneuro 5 (2018) ENEURO.0419-17.2018. https://doi.org/10.1523/ENEURO.0419-17.2018.

[31] B.A.A. Bus, M.L. Molendijk, B.J.W.H. Penninx, J.K. Buitelaar, G. Kenis, J. Prickaerts, B.M. Elzinga, R.C.O. Voshaar, Determinants of serum brain-derived neurotrophic factor, Psychoneuroendocrinology 36 (2011) 228-239. https://doi.org/10.1016/j .psyneuen.2010.07.013.

[32] M. Ventriglia, R. Zanardini, C. Bonomini, O. Zanetti, D. Volpe, P. Pasqualetti, M. Gennarelli, L. Bocchio-Chiavetto, Serum brain-derived neurotrophic factor levels in different neurological diseases, BioMed Res. Int. 2013 (2013) 901082. https://doi.org/10.1155/2013/901082.

[33] C. Yasutake, K. Kuroda, T. Yanagawa, T. Okamura, H. Yoneda, Serum BDNF, TNF-$\alpha$ and IL-1$\beta$ levels in dementia patients: Comparison between Alzheimer's disease and vascular dementia, Eur. Arch. Psychiatry Clin. Neurosci. 256 (2006) 402-406. https://doi.org/10.1007/s00406-006-0652-8.

[34] A.G. Ayankojo, R. Boroznjak, J. Reut, J. Tuvikene, T. Timmusk, V. Syritski, Electrochemical sensor based on molecularly imprinted polymer for rapid quantitative detection of brain-derived neurotrophic factor, Sens. Actuators B Chem. 397 (2023) 134656. https://doi.org/10.1016/j.snb.2023.134656.

[35] S. Alves, A. Paris, E. Rathahao-Paris, Mass spectrometry-based metabolomics for an in-depth questioning of human health, in: Adv. Clin. Chem., Elsevier, 2020: pp. 147-191. https://doi.org/10.1016/bs.acc.2020.02.009.

[36] A. Kidakova, R. Boroznjak, J. Reut, A. Öpik, M. Saarma, V. Syritski, Molecularly imprinted polymer-based SAW sensor for label-free detection of cerebral dopamine neurotrophic factor protein, Sens. Actuators B Chem. 308 (2020) 127708. https://doi.org/10.1016/j.snb.2020.127708.

## Claims

1. A method for a preparation of a molecularly-imprinted-polymer (MIP) electrochemical sensor for detection of electrochemically inactive analytes comprising at least one electrode with a redox-active surface, **characterized in that** said redox-active surface is preferably made of a transition metal oxide, and more preferably ruthenium oxide ($RuO_2$), wherein the redox-active surface is coated by a MIP layer, wherein the MIP layer comprises a plurality of analyte-selective cavities.

2. The method according to claim 1, comprising the steps where:

   in step (1) formation of a cleavable linking layer on the electrode,

   by a formation of a 3-(2-pyridyldithio) propionyl hydrazide (PDPH) monolayer on the redox-active surface ($RuO_2$) of the electrode; and
   by a formation of a 3,3'-dithiobis(sulfosuccinimidyl propionate) (DTSSP) monolayer covalently linked to the 3-(2-pyridyldithio) propionyl hydrazide (PDPH) monolayer;

   in step (2) immobilization of an electochemically inactive protein molecules on the cleavable linking layer;
   in step (3) polymerization of m-phenylenediamine (m-PD) on the electochemically inactive protein molecules immobilized onto the electrode thereby forming a polymeric layer coating on said electrically conductive surface with entrapped electochemically inactive protein molecules; and
   in step (4) cleavage of the cleavable linking layer thereby removing the electochemically inactive protein molecules from the polymeric layer and obtaining the MIP layer.

3. The method according to claim 2, where the electochemically inactive protein is preferably a neurotrophic factor, preferably brain-derived neurotrophic factor (BDNF).

4. A method for using a MIP electrochemical sensor **characterized by** comprising steps, wherein the electrochemical sensor is contacted with a sample solution comprising a target analyte for a defined time period, wherein the electrochemical sensor comprises at least one working electrode, a reference electrode and a counter electrode integrated on the insulative substrate, wherein a working electrode comprises a redox-active surface, wherein the redox-active surface is coated by a molecular imprinted polymer (MIP) layer, and wherein the target analyte selectively binds to a plurality of analyte-selective cavities within the MIP layer;

   where on the working electrode an electrochemical potential is imposed with the aid of a potentiostat;
   and an electrochemical current is measured with the aid of a potentiostat, wherein the electrochemical current is calibrated to a concentration of the target analyte in the sample solution.

5. The method of claim 4, wherein measuring the electrochemical current comprises measuring the electrochemical current by a voltametric method including, cyclic voltammetry, differential pulse voltammetry and more preferably linear sweep voltammetry, wherein the target analyte binding to the analyte-selective cavities of a MIP layer blocks a redox reaction at the redox-active electrode surface, and wherein the electrochemical current is recorded after an incubation period.

6. A molecularly-imprinted-polymer (MIP) electrochemical sensor for detection of electrochemically inactive analytes, comprising at least one electrode with an electrically conductive surface with a redox-active surface, preferably a transition metal oxide, and more preferably ruthenium oxide ($RuO_2$), wherein the redox-active surface is coated by a MIP layer, wherein the MIP layer comprises a plurality of analyte-selective cavities.

# FIG 1

FIG 2

FIG 3

LOD- 0.1 ng/mL
LOQ- 0.3 ng/mL

| Equation | y = a + b*x |
|----------|-------------|
| Intercept | 0 |
| Slope | 0.51 ± 0.01 |
| SD | 0.017 |
| R2 | 0.997 |

Conc of BDNF (ng/mL)

## FIG 4

FIG 5

FIG 6

Europäisches
Patentamt

European
Patent Office

Office européen
des brevets

**EUROPEAN SEARCH REPORT**

Application Number

EP 24 19 7428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 193 940 B2 (TALLINN UNIV OF TECHNOLOGY [EE]) 7 December 2021 (2021-12-07) * column 4, line 60 - column 35, line 32; claims 1-2; figures 1-5 * ----- | 1-6 | INV. G01N27/30 G01N33/543 |
| X | US 2023/408438 A1 (CHENG LI-JING [US] ET AL) 21 December 2023 (2023-12-21) * paragraphs [0032], [0036], [0058] - [0059], [0066] - [0094], [0108] - [0136]; figures 1-3,9-12 * ----- | 1,4-6 | |
| X | PHONKLAM KEWARIN ET AL: "A novel molecularly imprinted polymer PMB/MWCNTs sensor for highly-sensitive cardiac troponin T detection", SENSORS AND ACTUATORS B: CHEMICAL, ELSEVIER BV, NL, vol. 308, 25 December 2019 (2019-12-25), XP086017255, ISSN: 0925-4005, DOI: 10.1016/J.SNB.2019.127630 [retrieved on 2019-12-25] * abstract * * Sections 2.2 to 2.7; figure 1 * ----- | 1,4-6 | TECHNICAL FIELDS SEARCHED (IPC) G01N |
| X | US 2023/105411 A1 (GAO WEI [US] ET AL) 6 April 2023 (2023-04-06) * paragraphs [0007], [0016], [0032] - [0063]; figures 1-9 * ----- -/-- | 1,4-6 | |

The present search report has been drawn up for all claims

| Place of search | Date of completion of the search | Examiner |
|---|---|---|
| Munich | 10 February 2025 | Lazar, Zala |

EPO FORM 1503 03.82 (P04C01)

**page 1 of 2**

Europäisches
Patentamt

European
Patent Office

Office européen
des brevets

## EUROPEAN SEARCH REPORT

**Application Number**

EP 24 19 7428

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CAPOFERRI DENISE ET AL: "Electrochromic Molecular Imprinting Sensor for Visual and Smartphone-Based Detections", ANALYTICAL CHEMISTRY, vol. 90, no. 9, 1 May 2018 (2018-05-01), pages 5850-5856, XP093243517, ISSN: 0003-2700, DOI: 10.1021/acs.analchem.8b00389 * abstract * * Experimental section; figures 1-4 * | 1,4-6 | |
| X | STEPHY JOSE ET AL: "Electrochemical Glucose Sensing Using Molecularly Imprinted Polyaniline-Copper Oxide Coated Electrode", SURFACE ENGINEERING AND APPLIED ELECTROCHEMISTRY, ALLERTON PRESS, HEIDELBERG, vol. 58, no. 3, 1 June 2022 (2022-06-01), pages 260-268, XP037888897, ISSN: 1068-3755, DOI: 10.3103/S1068375522030127 [retrieved on 2022-06-27] * abstract * * Experimental methods * | 1,4-6 | |
| A | US 2012/097554 A1 (SHAH RAJIV [US] ET AL) 26 April 2012 (2012-04-26) * paragraph [0091] * | 1-6 | **TECHNICAL FIELDS SEARCHED (IPC)** |

The present search report has been drawn up for all claims

| Place of search | Date of completion of the search | Examiner |
|---|---|---|
| Munich | 10 February 2025 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 7428

This annex lists the patent family members relating to the patent documents cited in  the above-mentioned European search report.
The members are as contained in the European Patent Office EDP file on
The European Patent Office is in no way liable for these particulars which are merely given for the purpose of information.

10-02-2025

| Patent document cited in search report | | Publication date | Patent family member(s) | | Publication date |
|---|---|---|---|---|---|
| US 11193940 | B2 | 07-12-2021 | EE | 201900012 A | 16-11-2020 |
| | | | US | 2020319202 A1 | 08-10-2020 |
| US 2023408438 | A1 | 21-12-2023 | NONE | | |
| US 2023105411 | A1 | 06-04-2023 | CN | 113711040 A | 26-11-2021 |
| | | | EP | 3931568 A1 | 05-01-2022 |
| | | | US | 2020271639 A1 | 27-08-2020 |
| | | | US | 2023105411 A1 | 06-04-2023 |
| | | | WO | 2020176792 A1 | 03-09-2020 |
| US 2012097554 | A1 | 26-04-2012 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3721985 A1

- US 11193940 B2

**Non-patent literature cited in the description**

- Biological Biosensors for Monitoring and Diagnosis. **S. SINGH** ; **V. KUMAR** ; **D.S. DHANJAL** ; **S. DATTA** ; **R. PRASAD** ; **J. SINGH**. Microb. Biotechnol. Basic Res. Appl.. Springer Singapore, 2020, 317-335
- **A.G. AYANKOJO** ; **J. REUT** ; **V. SYRITSKI**. Electrochemically Synthesized MIP Sensors: Applications in Healthcare Diagnostics. *Biosensors*, 2024, vol. 14, 71, https://doi.org/10.3390/bios 14020071
- **A.G. AYANKOJO** ; **J. REUT** ; **V.B.C. NGUYEN** ; **R. BOROZNJAK** ; **V. SYRITSKI**. Advances in Detection of Antibiotic Pollutants in Aqueous Media Using Molecular Imprinting Technique-A Review. *Biosensors*, 2022, vol. 12, 441, https://doi.org/10.3390/-bios12070441
- **J.J. BELBRUNO**. Molecularly Imprinted Polymers. *Chem. Rev.*, 2019, vol. 119, 94-119, https://doi.org/10.1021/acs.chemrev.8b00171
- **D. ELFADIL** ; **A. LAMAOUI** ; **F. DELLA PELLE** ; **A. AMINE** ; **D. COMPAGNONE**. Molecularly Imprinted Polymers Combined with Electrochemical Sensors for Food Contaminants Analysis. *Molecules*, 2021, vol. 26, 4607, https://doi.org/10.3390/molecules26154607
- **P.S. SHARMA** ; **A. GARCIA-CRUZ** ; **M. CIEPLAK** ; **K.R. NOWORYTA** ; **W. KUTNER**. Gate effect' in molecularly imprinted polymers: the current state of understanding. *Curr. Opin. Electrochem.*, 2019, vol. 16, 50-56, https://doi.org/10.1016/j.coelec.2019.04.020
- **A.G. AYANKOJO** ; **J. REUT** ; **V. CIOCAN** ; **A. ÖPIK** ; **V. SYRITSKI**. Molecularly imprinted polymer-based sensor for electrochemical detection of erythromycin. *Talanta*, 2020, vol. 209, 120502, https://doi.org/10.1016/j.talanta.2019.120502
- **A.G. AYANKOJO** ; **R. BOROZNJAK** ; **J. REUT** ; **A. ÖPIK** ; **V. SYRITSKI**. Molecularly imprinted polymer based electrochemical sensor for quantitative detection of SARS-CoV-2 spike protein. *Sens. Actuators B Chem.,* 2022, vol. 353, 131160, https://doi.org/10.1016/j.snb.2021.131160

- **P. LACH** ; **M. CIEPLAK** ; **K.R. NOWORYTA** ; **P. PIETA** ; **W. LISOWSKI** ; **J. KALECKI** ; **R. CHITTA** ; **F. D'SOUZA** ; **W. KUTNER** ; **P.S. SHARMA**. Self-reporting molecularly imprinted polymer with the covalently immobilized ferrocene redox probe for selective electrochemical sensing of p-synephrine. *Sens. Actuators B Chem.*, 2021, vol. 344, 130276, https://doi.org/10.1016/j.snb.2021.130276
- **A. YARMAN** ; **F.W. SCHELLER**. How Reliable Is the Electrochemical Readout of MIP Sensors?. *Sensors*, 2020, vol. 20, 2677, https://doi.org/10.3390/s20092677
- **Z. SHI** ; **J. LI** ; **Y. WANG** ; **S. LIU** ; **J. ZHU** ; **J. YANG** ; **X. WANG** ; **J. NI** ; **Z. JIANG** ; **L. ZHANG**. Customized reaction route for ruthenium oxide towards stabilized water oxidation in high-performance PEM electrolyzers. *Nat. Commun.*, 2023, vol. 14, 843, https://doi.org/10.1038/s41467-023-36380-9
- **P.K. GUPTA** ; **L. MISHRA**. Ecofriendly ruthenium-containing nanomaterials: synthesis, characterization, electrochemistry, bioactivity and catalysis. *Nanoscale Adv.*, 2020, vol. 2, 1774-1791, https://doi.org/10.1039/D0NA00051E
- **M.R. AXET** ; **K. PHILIPPOT**. Catalysis with Colloidal Ruthenium Nanoparticles. *Chem. Rev.*, 2020, vol. 120, 1085-1145, https://doi.org/10.1021/acs.chemrev.9b00434
- **R.H.G. MINGELS** ; **S. KALSI** ; **Y. CHEONG** ; **H. MORGAN**. Iridium and Ruthenium oxide miniature pH sensors: Long-term performance, Sens. *Actuators B Chem.*, 2019, vol. 297, 126779, https://doi.org/10.1016/j.snb.2019.126779
- Ruthenium compounds: a new approach in nanochemistry. **SINGHA, P. P.** Ruthenium Chem.. Pan Stanford Publishing, 2018, 91-119
- **MISHRA, A. K.** ; **MISHRA, L.** Ruthenium chemistry. Pan Stanford Publishing, 2018
- **D. MAJUMDAR** ; **T. MAIYALAGAN** ; **Z. JIANG**. Recent Progress in Ruthenium Oxide-Based Composites for Supercapacitor Applications. *ChemElectroChem*, 2019, vol. 6, 4343-4372, https://doi.org/10.1002/celc.201900668

- **P. VEERAKUMAR** ; **S.-T. HUNG** ; **P.-Q. HUNG** ; **K.-C. LIN**. Review of the Design of Ruthenium-Based Nanomaterials and Their Sensing Applications in Electrochemistry. *J. Agric. Food Chem.*, 2022, vol. 70, 8523-8550, https://doi.org/10.1021/acs.jafc.2c01856

- **C.-C. KUNG** ; **P.-Y. LIN** ; **F.J. BUSE** ; **Y. XUE** ; **X. YU** ; **L. DAI** ; **C.-C. LIU**. Preparation and characterization of three dimensional graphene foam supported platinum-ruthenium bimetallic nanocatalysts for hydrogen peroxide based electrochemical biosensors. *Biosens. Bioelectron.*, 2014, vol. 52, 1-7, https://doi.org/10.1016/j.bios.2013.08.025

- **K.-J. CHEN** ; **K. CHANDRASEKARA PILLAI** ; **J. RICK** ; **C.-J. PAN** ; **S.-H. WANG** ; **C.-C. LIU** ; **B.-J. HWANG**. Bimetallic PtM (M=Pd, Ir) nanoparticle decorated multi-walled carbon nanotube enzyme-free, mediator-less amperometric sensor for H2O2. *Biosens. Bioelectron.*, 2012, vol. 33, 120-127, https://doi.org/10.1016/j.bios.2011.12.037

- **S.A. DELBARI** ; **L.S. GHADIMI** ; **R. HADI** ; **S. FARHOUDIAN** ; **M. NEDAEI** ; **A. BABAPOOR** ; **A. SABAHI NAMINI** ; **Q.V. LE** ; **M. SHOKOUHIMEHR** ; **M. SHAHEDI ASL**. Transition metal oxide-based electrode materials for flexible supercapacitors: A review. *J. Alloys Compd.*, 2021, vol. 857, 158281, https://doi.org/10.1016/j.jallcom.2020.158281

- **R. LIANG** ; **Y. DU** ; **P. XIAO** ; **J. CHENG** ; **S. YUAN** ; **Y. CHEN** ; **J. YUAN** ; **J. CHEN**. Transition Metal Oxide Electrode Materials for Supercapacitors: A Review of Recent Developments. *Nanomaterials*, 2021, vol. 11, 1248, https://doi.org/10.3390/nano11051248

- **A.G. AYANKOJO** ; **R. BOROZNJAK** ; **J. REUT** ; **J. TUVIKENE** ; **T. TIMMUSK** ; **V. SYRITSKI**. Electrochemical sensor based on molecularly imprinted polymer for rapid quantitative detection of brain-derived neurotrophic factor. *Sens. Actuators B Chem.*, 2023, vol. 397, 134656, https://doi.org/10.1016/j.snb.2023.134656

- **Y. PENG** ; **G. YI** ; **Z. GAO**. A highly sensitive microRNA biosensor based on ruthenium oxide nanoparticle-initiated polymerization of aniline. *Chem. Commun.*, 2010, vol. 46, 9131, https://doi.org/10.1039/c0cc01990a

- **Y. PENG** ; **Z. GAO**. Amplified Detection of MicroRNA Based on Ruthenium Oxide Nanoparticle-Initiated Deposition of an Insulating Film. *Anal. Chem.*, 2011, vol. 83, 820-827, https://doi.org/10.1021/ac102370s

- **B. CHAKRABORTY**. Electrochemical Properties of Sputtered Ruthenium Oxide Neural Stimulation and Recording Electrodes. *Electrochem*, 2023, vol. 4, 350-364, https://doi.org/10.3390/electrochem4030023

- **V.B.C. NGUYEN** ; **A.G. AYANKOJO** ; **J. REUT** ; **J. RAPPICH** ; **A. FURCHNER** ; **K. HINRICHS** ; **V. SYRITSKI**. Molecularly imprinted co-polymer for class-selective electrochemical detection of macrolide antibiotics in aqueous media. *Sens. Actuators B Chem.*, 2023, vol. 374, 132768, https://doi.org/10.1016/j.snb.2022.132768

- **M. ANTIPCHIK** ; **J. REUT** ; **A.G. AYANKOJO** ; **A. ÖPIK** ; **V. SYRITSKI**. MIP-based electrochemical sensor for direct detection of hepatitis C virus via E2 envelope protein. *Talanta*, 2022, vol. 250, 123737, https://doi.org/10.1016/j.talanta.2022.123737

- **A.G. AYANKOJO** ; **J. REUT** ; **A. ÖPIK** ; **V. SYRITSKI**. Sulfamethizole-imprinted polymer on screen-printed electrodes: Towards the design of a portable environmental sensor. *Sens. Actuators B Chem.*, 2020, vol. 320, 128600, https://doi.org/10.1016/j.snb.2020.128600

- **Y. NAEGELIN** ; **H. DINGSDALE** ; **K. SÄUBERLI** ; **S. SCHÄDELIN** ; **L. KAPPOS** ; **Y.-A. BARDE**. Measuring and Validating the Levels of Brain-Derived Neurotrophic Factor in Human Serum. *Eneuro*, 2018, vol. 5, https://doi.org/10.1523/ENEURO.0419-17.2018

- **B.A.A. BUS** ; **M.L. MOLENDIJK** ; **B.J.W.H. PENNINX** ; **J.K. BUITELAAR** ; **G. KENIS** ; **J. PRICKAERTS** ; **B.M. ELZINGA** ; **R.C.O. VOSHAAR**. Determinants of serum brain-derived neurotrophic factor. *Psychoneuroendocrinology*, 2011, vol. 36, 228-239, https://doi.org/10.1016/j.psyneuen.2010.07.013

- **M. VENTRIGLIA** ; **R. ZANARDINI** ; **C. BONOMINI** ; **O. ZANETTI** ; **D. VOLPE** ; **P. PASQUALETTI** ; **M. GENNARELLI** ; **L. BOCCHIO-CHIAVETTO**. Serum brain-derived neurotrophic factor levels in different neurological diseases. *BioMed Res. Int.*, 2013, vol. 2013, 901082, https://doi.org/10.1155/2013/901082

- **C. YASUTAKE** ; **K. KURODA** ; **T. YANAGAWA** ; **T. OKAMURA** ; **H. YONEDA** ; **SERUM BDNF**. TNF-$\alpha$ and IL-1$\beta$ levels in dementia patients: Comparison between Alzheimer's disease and vascular dementia. *Eur. Arch. Psychiatry Clin. Neurosci.*, 2006, vol. 256, 402-406, https://doi.org/10.1007/s00406-006-0652-8

- Mass spectrometry-based metabolomics for an in-depth questioning of human health. **S. ALVES** ; **A. PARIS** ; **E. RATHAHAO-PARIS**. Clin. Chem.. Elsevier, 2020, 147-191

- **A. KIDAKOVA** ; **R. BOROZNJAK** ; **J. REUT** ; **A. ÖPIK** ; **M. SAARMA** ; **V. SYRITSKI**. Molecularly imprinted polymer-based SAW sensor for label-free detection of cerebral dopamine neurotrophic factor protein. *Sens. Actuators B Chem.*, 2020, vol. 308, 127708, https://doi.org/10.1016/j.snb.2020.127708